# FASCICULE DE BREVET EUROPEEN

(11) **EP 1 296 627 B1**
(45) Date de publication et mention de la délivrance du brevet: **28.04.2004**
(21) Numéro de dépôt: 01969345.6
(22) Date de dépôt: 06.07.2001
(51) Int. Cl.: A61F 15/00, A45D 34/04

(54) **EMBALLAGE A POCHETTES MULTIPLES POUR UN SUPPORT IMPREGNE D'UN PRODUIT POUR APPLICATION CUTANEE ET POUR DES MOYENS DE PROTECTION DE LA ZONE CUTANEE TRAITEE**
VERPACKUNG MIT MEHREREN BEUTELN FÜR EINEN MIT EINEM HAUTPFLEGEMITTEL GETRÄNKTEN TRÄGER UND FÜR HAUTSCHUTZMITTEL
MULTIPLE-POUCH PACKAGE FOR A SUPPORT IMPREGNATED WITH A PRODUCT FOR APPLICATION ON THE SKIN AND FOR MEANS PROTECTING THE TREATED SKIN ZONE

(30) Priorité: 06.07.2000 FR 0008869
(43) Date de publication de la demande: 02.04.2003
(73) Titulaire: Wendel, Serge, 68340 Riquewihr (FR); Dos Santos, Wilton, 67200 Strasbourg (FR)
(72) Inventeur: Wendel, Serge, 68340 Riquewihr (FR); Dos Santos, Wilton, 67200 Strasbourg (FR)
(74) Mandataire: Vièl, Frédérique
(86) Numéro de dépôt international: PCT/EP2001/007773
(87) Numéro de publication internationale: WO 2002/002042

(56) Documents cités:
- WO-A-85/03275
- FR-A- 1 408 584
- US-A- 4 304 333
- US-A- 4 360 020
- US-A- 5 487 932

## Description

L'invention concerne un emballage pour support imprégné d'un produit pour application cutanée, comprenant une première pochette constituée essentiellement de deux parois fixées l'une à l'autre par des moyens de fixation appropriés permettant, au moment de l'utilisation, de désolidariser au moins partiellement lesdites parois, ladite pochette étant destinée à contenir un support imprégné du produit à appliquer, le support imbibé étant fixé par des moyens appropriés sur la face interne d'une au moins des parois de la première pochette.

Il est courant d'utiliser en pharmacie, en cosmétique ou en parfumerie des supports imprégnés d'un produit pour application cutanée présentés dans des pochettes pour usage unique. La pochette est ouverte et le support imprégné du produit à appliquer est sorti pour ensuite être apposé sur la partie de la peau choisie. Ces pochettes à usage unique sont souvent employées comme objet publicitaire, en voyage ou bien encore dans le domaine de la pharmacie, lorsque l'usage d'une dose unique est privilégié, par exemple lorsque la stérilité du produit à appliquer doit être assurée.

De telles pochettes sont décrites notamment dans les documents WO 98 / 56 568 ou EP 0 953 303. Un inconvénient majeur de ces pochettes réside dans le fait qu'il faille saisir le support imbibé pour appliquer le produit sur la partie de la peau choisie. Le produit à appliquer est alors déposé non seulement sur la partie de la peau choisie mais également sur les doigts manipulant le support imbibé. Ceci peut s'avérer très gênant, par exemple en raison de la couleur du produit appliqué, par exemple l'éosine en pharmacie, ce qui nécessite de se laver soigneusement les mains après application. Par ailleurs, il est courant que cette opération ait lieu dans des conditions d'hygiène incertaine. Ce sera notamment le cas des lingettes de désinfectant ou de cicatrisant utilisées lors d'une excursion en l'absence de point d'eau pour se laver préalablement les mains. Si le produit à appliquer est présenté dans une enveloppe assurant sa stérilité lors du stockage, cette stérilité n'est plus assurée et le support imbibé est souillé par les doigts au risque de souiller ensuite la plaie et de provoquer une infection.

Pour remédier à cet inconvénient, des pochettes ont été proposées dans lesquelles le support imprégné est fixé sur une au moins des parois de la pochette. De telles pochettes sont connues de US 5 487 932 A, US 4 360 020 A, WO 85 03 275 A et FR 1 408 584 A.

De plus, US 4 360 020 présente un emballage à double pochette, chaque pochette possédant un support imprégné.

Dans le domaine des premiers secours cependant, il est parfois nécessaire d'avoir non seulement un support imprégné d'une solution pour application locale, tel qu'un désinfectant ou un cicatrisant, mais également de quoi protéger la zone ainsi traitée. Il faut donc penser à ranger ensemble ou à emmener d'une part de quoi traiter la plaie et d'autre part de quoi la protéger. Durant les randonnées ou en voyage par exemple, le risque est grand d'égarer dans un sac l'un ou l'autre élément. Par ailleurs, l'utilisation de plusieurs emballages augmente la quantité d'éléments à jeter, au risque d'en oublier quelques uns au bord du chemin.

L'invention a donc pour objet de développer un emballage du genre de celui décrit dans le préambule qui permette facilement de trouver les différents éléments nécessaires au traitement et à la protection d'une zone cutanée.

Cet objectif est atteint par un emballage du genre présenté précédemment et qui comporte au moins une pochette supplémentaire constituée essentiellement de deux parois fixées l'une à l'autre par des moyens de fixation appropriés permettant, au moment de l'utilisation, de désolidariser au moins partiellement lesdites parois et destinée à recevoir des moyens de protection de la zone cutanée traitée. Ainsi, un même emballage comprend dans deux pochettes différentes d'une part un support imprégné et d'autre part des moyens de protection de la zone traitée. Les moyens de protection peuvent être constitués d'un bandage, d'une compresse, d'un sparadrap ou d'un pansement.

Pour faciliter l'application du produit, il est préférable que la ou chaque paroi de la première pochette sur laquelle est fixé le support imbibé soit confectionnée dans un matériau souple. En effet, il sera plus facile d'appliquer le produit dans les zones de la peau en retrait ou en saillie.

Pour optimiser la forme et le stockage des emballages conformes à l'invention, il est prévu de superposer au moins deux pochettes l'une sur l'autre de sorte que deux pochettes successives sont reliées par une paroi commune. Cette paroi commune pourra être formée en fixant par des moyens appropriés les faces extérieures des parois de deux pochettes, c'est-à-dire en fixant l'une sur l'autre deux pochettes individuelles. Elle pourra également être obtenue en utilisant les deux faces d'une paroi unique de sorte à obtenir d'un côté la face interne de la première pochette et de l'autre côté la face interne de la pochette suivante.

Ces pochettes supplémentaires sont prévues principalement pour recevoir, selon la destination de l'emballage, un bandage, une compresse, un sparadrap, un pansement ou un support supplémentaire imprégné du même produit ou d'un autre produit, la compresse ou le support imprégné supplémentaire pouvant être fixé sur l'une au moins des parois de ladite pochette. On pourra ainsi combiner un désinfectant et/ou un cicatrisant avec un pansement.

Des moyens peuvent être prévus, dans les pochettes destinées plus particulièrement aux produits pharmaceutiques stériles, pour garantir la stérilité du contenu du produit durant le stockage. On choisira par exemple pour fabriquer les parois de ces pochettes un matériau apte à garantir la stérilité du produit, comme par exemple des couches de laminé ou de complexe de laminé. La fixation des parois sera alors assurée par exemple par soudure, collage ou thermoscellage.

Afin de faciliter l'ouverture des pochettes, il est préférable que les moyens de fixation des parois de chaque pochette permettent une séparation par pelage desdites parois lors de l'ouverture de la pochette considérée.

Il est conforme à l'invention de prévoir des moyens antirupture au moins sur la première pochette pour empêcher, lors de l'ouverture de la pochette considérée, que les parois qui la forment ne se désolidarisent totalement. Les moyens antirupture des pochettes contenant un support imprégné peuvent être constitués notamment par la fixation dudit support imprégné sur les deux parois de la pochette le contenant. Ainsi, lorsque le support imbibé, plié en deux, est fixé sur les deux parois de la pochette, il empêche par sa résistance propre la séparation totale des parois. Une autre solution consiste à fixer les deux parois sur une certaine section par un mode de fixation beaucoup plus résistant que sur le reste de la pochette. Si la pochette est formée par deux pellicules rectangulaires fixées l'une sur l'autre par exemple par soudage, trois des soudures s'ouvriront facilement par simple pelage tandis que la quatrième soudure résistera à l'arrachement, par exemple grâce au support imprégné fixé sur les deux parois ou à un autre type de soudure plus résistant. Dans ce dernier cas, cette soudure plus résistante peut servir de poignée facilitant le maniement du support imprégné. Lors de l'utilisation, la première pochette est ouverte, la ou les parois sur lesquelles est fixé le support imprégné est saisie par l'utilisateur pour appliquer le produit. Lorsque l'application est finie, la paroi libre de la première pochette est rabattue pour couvrir le support imprégné et la seconde pochette est ouverte pour accéder à son contenu. En munissant les deux pochettes de tels moyens antirupture, on diminue le nombre de pièces à jeter après utilisation, évitant ainsi d'égarer ou de laisser s'envoler des morceaux d'emballage qui risqueraient de polluer l'environnement.

Afin de faciliter l'ouverture de chaque pochette, celles-ci peuvent comporter des moyens d'ouverture. Des languettes, formant le prolongement des parois de la pochette au-delà d'une des soudures, pourront servir par exemple de moyens d'ouverture.

Lorsque plusieurs pochettes superposées comportent de tels moyens antirupture et ou d'ouverture, il est préférable que les moyens antirupture et / ou les moyens d'ouverture d'une pochette soient placés, sur la paroi commune aux deux pochettes, décalés ou à l'opposé par rapports respectivement aux moyens antirupture et / ou aux moyens d'ouverture de la pochette suivante. En plaçant ces moyens d'ouverture décalés ou à l'opposé les uns par rapport aux autres, on évite qu'une pochette ne soit ouverte par mégarde lors de l'ouverture d'une autre pochette.

Quelques exemples de réalisation de l'invention sont présentés ci-dessous à l'aide des figures suivantes :
- Figure 1: Première pochette contenant un support imprégné fixé sur une seule paroi de la pochette ;
- Figure 2: La première pochette de la figure 1 après ouverture ;
- Figure 3: Première pochette, ouverte, contenant un support imprégné fixé sur les deux parois de la pochette ;
- Figure 4: Emballage à deux pochettes, fermé, contenant un support imprégné fixé aux deux parois de la première pochette et un pansement dans la seconde pochette ;
- Figure 5: Emballage à trois pochettes, fermé, contenant deux supports imprégnés et un pansement ;
- Figure 6: Utilisateur ouvrant un emballage à deux pochettes.

L'emballage selon l'invention comporte une première pochette (3, 11, 31) dans laquelle est fixée un support imprégné (2, 14, 32) et au moins une seconde pochette (17, 39) contenant des moyens de protection de la zone cutanée traitée, tels qu'un sparadrap, un bandage, une compresse ou un pansement (22, 40).

Les pochettes peuvent être constituées de différentes façons.

Dans un premier mode de réalisation, la pochette (3) se compose d'une bandelette (1) souple en laminé ou complexe de laminé. La bandelette (1) est repliée dans son milieu de sorte à enfermer le support imprégné (2) ou les moyens de protection de la plaie dans une sorte de pochette (3). La pochette (3) est fermée sur ses bords en fixant l'une sur l'autre les deux demi-bandelettes par soudure, collage ou thermoscellage sur la périphérie (4) de la bandelette (1). Les deux demi-bandelettes forment alors les parois de la pochette (3).

Dans un second mode de réalisation, la pochette (11, 17, 31, 35, 39) est réalisée en utilisant deux bandelettes (12, 13, 18, 19, 33, 34, 37, 38) fixées l'une sur l'autre sur toute la périphérie.

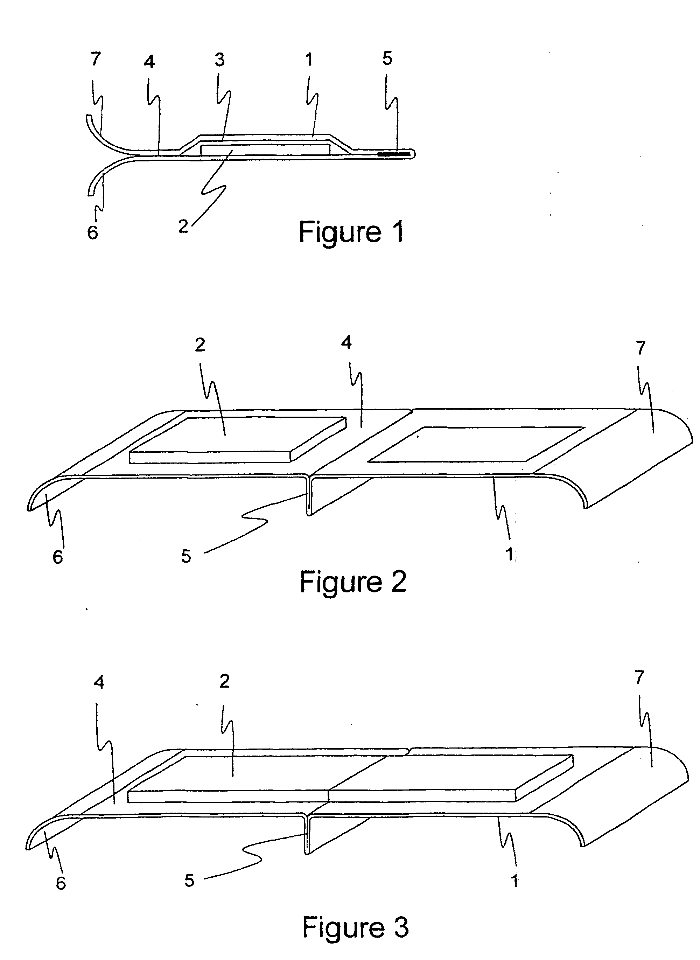

Le support imprégné (2, 14, 32) est fixé dans la première pochette (3, 11, 31) par exemple par soudure. Le support imprégné (2, 14, 32) peut être fabriqué dans un matériau synthétique ou semi-synthétique. Le matériau du support imprégné (2, 14, 32) est choisi pour ses capacités d'absorption du produit à appliquer. Le support imprégné (2, 14, 32) peut être fixé soit sur une seule paroi de la pochette comme le montrent les figures 1 et 2, soit sur les deux parois comme le montrent les figures 3, 4 et 5.

Pour éviter la prolifération de pièces à jeter lors de l'utilisation, il est préférable que les différentes pochettes (3, 11, 17, 31, 35, 39) soient munies de moyens antirupture (5) qui évitent que les deux parois d'une pochette ne soient entièrement séparées l'une de l'autre. Ainsi, dans l'exemple montré à la figure 2, la paroi libre reste accrochée à l'emballage par une fixation plus résistante (marquée en gras sur les figure 1), de sorte qu'à la fin de l'utilisation, il n'y a qu'une pièce à jeter. De plus, cette paroi peut être rabattue sur le support imprégné afin d'éviter que les restes de produit à appliquer ne soient appliqués par inadvertance après utilisation. Dans les exemples des figures 3, 4 et 5, ces moyens antirupture sont constitués par le support imprégné (2, 14, 32) lui-même qui est fixé d'une pièce sur les deux parois.

Par ailleurs, en fixant le support imprégné (2) non pas au niveau de la pliure de la bandelette (1) mais à une certaine distance de celle-ci et en fixant les deux demi-bandelettes l'une sur l'autre par exemple par soudure sur cette portion située au-delà de la pliure du support imprégné, il se forme une sorte de poignée (5) qui facilite le maniement du support imprégné (2).

Pour faciliter l'ouverture des pochettes, il est possible de former deux languettes (6, 7, 15, 16, 20, 21, 41, 42, 43, 44, 45) de part et d'autre de la soudure d'ouverture, de préférence à l'opposé des moyens antirupture. La forme générale rectangulaire n'est pas une obligation. On pourra par exemple choisir une forme circulaire, notamment lorsque le support imprégné n'est fixé que sur une paroi de la pochette.

L'emballage selon l'invention peut être obtenu en superposant au moins deux pochettes simples du type montré aux figures 1 à 3.

Pour cela, on pourra soit fixer des pochettes simples les unes aux autres, soit replier en accordéon une bandelette plusieurs fois sur elle-même pour former des pochettes successives dont les bords périphériques seront par exemple soudés.

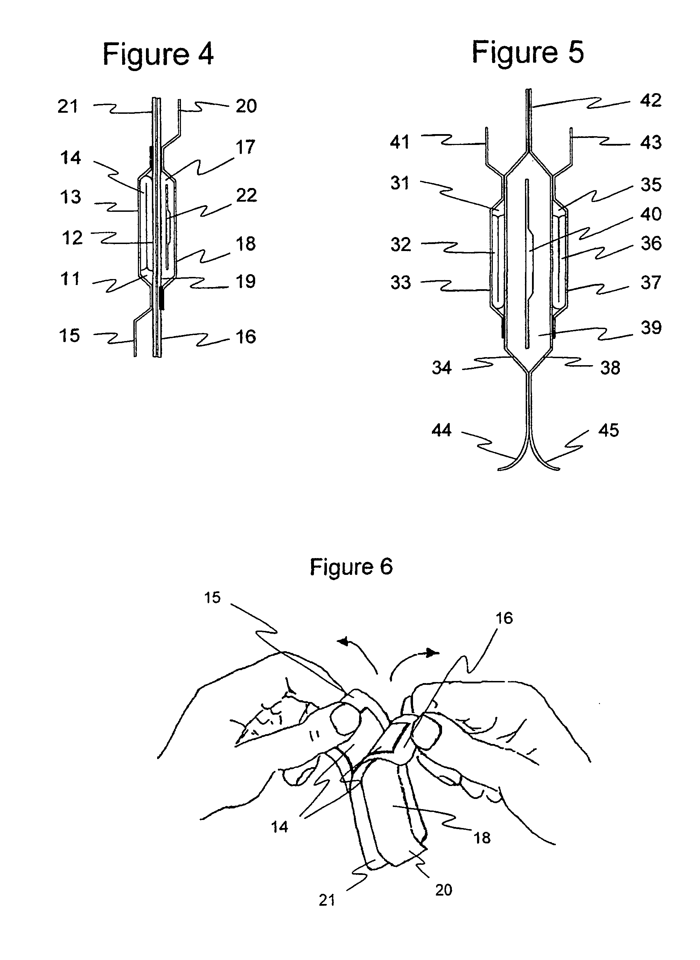

L'exemple de la figure 4 comporte une première pochette (11) formée par deux bandelettes (12, 13) soudées l'une sur l'autre au niveau de leurs bords périphériques, formant ainsi les deux parois principales de la première pochette (11). Un support imprégné (14) est fixé sur les deux parois (12, 13) de la première pochette (11). Lorsque l'utilisateur tire, comme le montre la figure 6, sur les languettes (15, 16) formées par les extrémités des bandelettes (12, 13), il ouvre la pochette par pelage. En rabattant la bandelette extérieure (13), le support imprégné (14) est déplié et le produit peut être appliqué. Le support imprégné (14) est fixé sur les parois (12, 13) de la pochette (11) de sorte qu'elles ne peuvent pas se désolidariser. Si le support imprégné (14) n'était fixé que sur une des parois, par exemple la paroi centrale (12), il serait possible de fixer la paroi extérieure (13) sur la paroi centrale (12) au niveau d'un de ses bords périphériques par une soudure plus résistante, afin d'éviter que la paroi extérieure (13) ne se désolidarise du reste de l'emballage. Cette soudure résistante remplirait alors la fonction de moyens antirupture.

Une seconde pochette (17), formée par deux bandelettes (18, 19) soudées ensemble, est fixée sur la paroi (12) de la première pochette (11). Des moyens pour faciliter l'ouverture de cette seconde pochette (17) sont également prévus, sous forme de languettes (20, 21) constituées par les extrémités des bandelettes (18, 19) formant la seconde pochette (17). La fixation de la bandelette extérieure (18) sur la bandelette centrale (19) sera faite de façon à permettre une séparation au moins partielle des parois par pelage. La fixation située à l'opposé des languettes (20, 21) sera de préférence plus résistante, afin d'éviter que la paroi (18) ne se sépare complètement du reste de l'emballage. Les bandelettes (12, 19) forment une paroi commune aux deux pochettes.

La seconde pochette contient des moyens de protection de la zone cutanée traitée, par exemple un sparadrap, un pansement (22), une compresse ou encore un bandage.

Une pochette de ce genre peut également être obtenue en repliant en accordéon une même bandelette deux fois ou en fixant deux bandelettes extérieures sur une bandelette centrale formant la paroi commune.

On positionnera les moyens pour faciliter l'ouverture (15, 16, 20, 21) des deux pochettes (11, 17) à l'opposé les uns des autres, grâce à quoi on évite qu'une pochette soit ouverte à la place de l'autre.

La figure 5 montre un autre exemple d'emballage comportant trois pochettes. La première pochette (31) contient un support imprégné (32) fixé à deux parois (33, 34), une pochette (35) supplémentaire contient un second support imprégné (36) fixé à deux parois (37, 38) et la seconde pochette (39) formée par les faces externes des parois (34, 38) des deux autres pochettes contient les moyens de protection de la zone cutanée traitée, par exemple un sparadrap, un pansement (40), un bandage ou une compresse. Cet emballage peut servir de kit de premier secours. L'utilisateur ouvre la première pochette (31) en écartant les languettes (41, 42) pour pouvoir déplier le premier support imprégné (32) par exemple de désinfectant. Une fois la plaie désinfectée, l'utilisateur rabat la paroi (33) et ouvre la pochette supplémentaire (35) pour accéder au second support imprégné (36) par exemple de cicatrisant. Pour faciliter l'ouverture de la pochette (35) supplémentaire, celle-ci présente également deux languettes (42, 43). Enfin, le pansement (40) contenu dans la seconde pochette (39) peut être libéré en ouvrant cette dernière pochette à l'aide des languettes (44, 45). Une fois encore, il est préférable que cette seconde pochette soit munie de moyens antirupture, par exemple en soudant ses deux parois de façon plus résistance du côté opposé aux languettes (44, 45). A la fin de l'opération, tous les composants de l'emballage sont solidaires les uns des autres, ce qui fait qu'il n'y a qu'une pièce à jeter. On ne risque donc pas de laisser traîner des restes du kit de premiers secours.

En positionnant les languettes comme le montrent les figures 4, 5 et 6, on facilite l'ouverture successive des différentes pochettes, sans risquer d'ouvrir une pochette à la place d'une autre.

Bien que les emballages cités ici à titre d'exemple ne comportent que deux ou trois pochettes, il va de soi que ce nombre n'est pas limité et qu'il est possible de réaliser des emballages avec un nombre de pochettes plus important selon l'usage envisagé.

Il est également possible de placer les moyens antirupture ou les moyens pour faciliter l'ouverture des pochettes successives non pas à l'opposé les uns des autres mais de façon décalée. Ce sera par exemple le cas, si on choisit un emballage à trois pochettes de forme triangulaire. Les moyens antirupture seront positionnés chacun sur un bord du triangle et les moyens pour faciliter l'ouverture sur l'angle opposé.

Au lieu de superposer les différentes pochettes les unes sur les autres, il est également possible de les aligner. Un emballage à deux pochettes peut être par exemple formé d'une bandelette inférieure allongée sur laquelle seront fixées deux bandelettes supérieures de dimensions moindres pour former deux pochettes côte à côte. Ces pochettes pourront être munies comme dans les exemples présentés précédemment de moyens antirupture formés par exemple par des fixations plus résistantes ou par le support imprégné fixé sur les deux parois ainsi que de moyens facilitant l'ouverture, par exemple sous forme de languettes, qui pourront être positionnés décalés les uns par rapport aux autres.

La composition des bandelettes formant les parois dépendra de l'usage fait des pochettes. Les pochettes contenant un produit liquide ou semi-solide seront réalisées dans des matériaux étanches compatibles avec le produit contenu, par exemple en laminé ou complexe de laminé de polyester / aluminium / polyéthylène. Le cas échéant, le matériau devra assurer la stérilité du produit contenu dans la pochette. Pour les pochettes contenant les sparadrap, les compresses, des pansements ou les bandages, il sera possible d'utiliser des bandelettes en papier avec un vernis acrylique. Le support imprégné pourra par exemple être réalisé dans une mousse de polyester. En réalisant les emballages à pochettes multiples par fixation de différentes pochettes simples les unes sur les autres, il est possible d'utiliser différents matériaux pour les différents contenus.

L'emballage selon l'invention présente donc de nombreux avantages par rapport aux emballages connus. L'avantage principal réside dans le fait que le même emballage contient le produit pour application cutanée et les moyens de protection de la zone cutanée ainsi traitée. On ne risque donc pas d'oublier l'un ou l'autre ou d'égarer l'un de ces éléments.

Les différents éléments formant l'emballage pouvant être rendus solidaires les uns des autres par des moyens antirupture, l'utilisateur a tout au long des différentes étapes d'utilisation qu'une seule pièce à manipuler et finalement à jeter.

### Liste des références

- 1: Bandelette
- 2: Support imprégné
- 3: Pochette
- 4: Périphérie de la pochette
- 5: Poignée
- 6: Languette
- 7: Languette
- 11: Pochette
- 12: Bandelette
- 13: Bandelette
- 14: Support imprégné
- 15: Languette
- 16: Languette
- 17: Pochette
- 18: Bandelette
- 19: Bandelette
- 20: Languette
- 21: Languette
- 22: Pansement
- 31: Pochette
- 32: Support imprégné
- 33: Bandelette
- 34: Bandelette
- 35: Pochette
- 36: Support imprégné
- 37: Bandelette
- 38: Bandelette
- 39: Pochette
- 40: Pansement
- 41: Languette
- 42: Languette
- 43: Languette
- 44: Languette
- 45: Languette

## Revendications

1. Emballage pour support imprégné d'un produit pour application cutanée, comprenant une première pochette (3, 11, 31) constituée essentiellement de deux parois (1, 12, 13, 33, 34) fixées l'une à l'autre par des moyens de fixation appropriés permettant, au moment de l'utilisation, de désolidariser au moins partiellement lesdites parois (1, 12, 13, 33, 34), ladite pochette (3, 11, 31) contenant un support imprégné (2, 14, 32) du produit à appliquer, le support imbibé (2, 14, 32) étant fixé par des moyens appropriés sur la face interne d'une au moins des parois (1, 12, 13. 33, 34) de la première pochette (3, 11, 31), **caractérisé en ce qu**'il comporte au moins une seconde pochette (17, 39) constituée essentiellement de deux parois (18, 19, 34, 38) fixées l'une à l'autre par des moyens de fixation appropriés permettant, au moment de l'utilisation, de désolidariser au moins partiellement lesdites parois et contenant des moyens de protection (22, 40) de la zone cutanée traitée.

2. Emballage selon la revendication 1, **caractérisé en ce que** les moyens de protection de la zone cutanée traitée sont constitués d'un bandage, d'une compresse, d'un sparadrap ou d'un pansement (22, 40).

3. Emballage selon la revendication 1, **caractérisé en ce que** la ou chaque paroi (1, 12, 13, 33, 34) de la première pochette (3, 11, 31) sur laquelle est fixé le support imprégné (2, 14, 32) est confectionnée dans un matériau souple.

4. Emballage selon la revendication 1, **caractérisé en ce que** deux pochettes (11, 17, 31, 35, 39) au moins sont superposées l'une sur l'autre de sorte que deux pochettes successives soient reliées par une paroi commune (12, 34, 38).

5. Emballage selon la revendication 1, **caractérisé en ce que** des moyens sont prévus dans l'une ou les pochettes destinées à recevoir un contenu stérile pour assurer la stérilité dudit contenu lors du stockage.

6. Emballage selon la revendication 1, **caractérisé en ce que** les moyens de fixation des parois (1, 12, 13, 18, 33, 34, 37, 38) de chaque pochette (3, 11, 17, 31, 35, 39) permettent une séparation par pelage desdites parois lors de l'ouverture de la pochette considérée.

7. Emballage selon la revendication 1, **caractérisé en ce que** des moyens antirupture (2, 14, 32, 36) sont prévus au moins sur la première pochette (3, 11, 31) pour empêcher, lors de l'ouverture de la pochette considérée, que les parois (1, 12, 13, 33, 34) qui la forment ne se désolidarisent totalement.

8. Emballage selon la revendication précédente, **caractérisé en ce que** les moyens antirupture des pochettes (3, 11, 31, 35) contenant un support imprégné (2, 14, 32, 36) sont constitués notamment par la fixation dudit support imprégné sur les deux parois de la pochette le contenant.

9. Emballage selon la revendication 1, **caractérisé en ce que** l'une ou chaque pochette (3, 11, 17, 31, 35, 39) comporte des moyens d'ouverture (6, 7, 15, 16, 20, 21, 41, 42, 43, 44, 45) pour faciliter son ouverture.

10. Emballage selon les revendications 7 à 9, **caractérisé en ce que** les moyens antirupture et / ou les moyens d'ouverture (6, 7, 15, 16, 20, 21, 41, 42, 43, 44, 45) d'une pochette sont placés décalés ou à l'opposé par rapport respectivement aux moyens antirupture et / ou aux moyens d'ouverture de la pochette suivante, sur la paroi commune aux deux pochettes.

## Patentansprüche

1. Verpackung für mit einem Produkt zur kutanen Anwendung durchfeuchtetes Trägermaterial, welche ein erstes Täschchen (3, 11, 31) umfasst, welches im wesentlichen aus zwei Wänden (1, 12, 13, 33, 34) gebildet ist, die aneinander durch geeignete Befestigungsmittel, die es bei der Verwendung ermöglichen, wenigstens teilweise die Wände (1, 12, 13, 33, 34) zu trennen, befestigt sind, wobei das Täschchen (3, 11, 31) ein mit einem anzuwendenden Produkt durchfeuchtetes Trägermaterial (2, 14, 32) enthält, wobei das durchtränkte Trägermaterial (2, 14, 32) durch geeignete Mittel an der Innenseite wenigstens einer der Wände (1, 12, 13, 33, 34) des ersten Täschchens (3, 11, 31) befestigt ist, **dadurch gekennzeichnet, dass** sie wenigstens ein zweites Täschchen (17, 39) umfasst, welches im wesentlichen aus zwei Wänden (18, 19, 34, 38) gebildet ist, die aneinander durch geeignete Befestigungsmittel, die es bei der Verwendung ermöglichen, wenigstens teilweise die Wände zu trennen, befestigt sind, und Mittel zum Schutz (22, 40) des behandelten kutanen Bereichs enthält.

2. Verpackung gemäß Anspruch 1, **dadurch gekennzeichnet, dass** die Mittel zum Schutz des behandelten kutanen Bereichs gebildet sind aus einer Binde, einer Kompresse, einem Heftpflaster oder einem Pflaster (22, 40).

3. Verpackung gemäß Anspruch 1, **dadurch gekennzeichnet, dass** die oder jede Wand (1, 12, 13, 33, 34) des ersten Täschchens (3, 11, 31), auf welcher das durchfeuchtete Trägermaterial (2, 14, 32) befestigt ist, aus einem weichen Material hergestellt ist.

4. Verpackung gemäß Anspruch 1, **dadurch gekennzeichnet, dass** wenigstens zwei Täschchen (11, 17, 31, 35, 39) übereinander gelegt sind, so dass zwei aufeinanderfolgende Täschchen durch eine gemeinsame Wand (12, 34, 38) verbunden sind.

5. Verpackung gemäß Anspruch 1, **dadurch gekennzeichnet, dass** in einem oder den zum Aufnehmen eines sterilen Inhalts bestimmten Täschchen Mittel vorgesehen sind, um die Sterilität des Inhalts bei der Lagerung sicherzustellen.

6. Verpackung gemäß Anspruch 1, **dadurch gekennzeichnet, dass** die Befestigungsmittel der Wände (1, 12, 13, 18, 33, 34, 37, 38) jedes Täschchens (3, 11, 17, 31, 35, 39) ein Trennen durch Abziehen der Wände bei Öffnen des in Betracht gezogenen Täschchens ermöglichen.

7. Verpackung gemäß Anspruch 1, **dadurch gekennzeichnet, dass** Mittel gegen Zerreißen (2, 14, 32, 35) wenigstens an dem ersten Täschchen (3, 11, 31) vorgesehen sind, um bei Öffnen des in Betracht gezogenen Täschchens zu verhindern, dass die Wände (1, 12, 13, 33, 34), die dieses bilden, sich vollständig voneinander trennen.

8. Verpackung gemäß dem vorausgegangenen Anspruch, **dadurch gekennzeichnet, dass** die Mittel gegen Zerreißen der ein durchfeuchtetes Trägermaterial (2, 14, 32, 36) enthaltenden Täschchen (3, 11, 31, 35) insbesondere gebildet sind durch Befestigung des durchfeuchteten Trägermaterials an den beiden Wänden des dieses enthaltenden Täschchens.

9. Verpackung gemäß Anspruch 1, **dadurch gekennzeichnet, dass** ein oder jedes Täschchen (3, 11, 17, 31, 35, 39) Mittel zum Öffnen (6, 7, 15, 16, 20, 21, 41, 42, 43, 44, 45) umfasst, um sein Öffnen zu erleichtern.

10. Verpackung gemäß den Ansprüchen 7 bis 9, **dadurch gekennzeichnet, dass** die Mittel gegen Zerreißen und / oder die Mittel zum Öffnen (6, 7, 15, 16, 20, 21, 41, 42, 43, 44, 45) eines Täschchens versetzt oder gegenüber liegend angeordnet sind im Hinblick jeweils auf die Mittel gegen Zerreißen und / oder die Mittel zum Öffnen des folgenden Täschchens, auf der den beiden Täschchen gemeinsamen Wand.

## Claims

1. A packaging for a support impregnated with a produce for cutaneous application, comprising a first pocket (3, 11, 31) consisting essentially of two walls (1, 12, 13, 33, 34) secured to one another by appropriate fastening means enabling, during usage, to disconnect at least partially said walls (1, 12, 13, 33, 34), said pocket (3, 11, 31) containing a support (2, 14, 32) impregnated with the produce to be applied, the soaked support (2, 14, 32) being secured by appropriate means to the inner face of at least one of the walls (1, 12, 13, 33, 34) of the first pocket (3, 11, 31 ), **characterised in that** it comprises at least a second pocket (17, 39) consisting essentially of two walls (18, 19, 34, 38) secured to one another by appropriate fastening means enabling, during usage, to disconnect at least partially said walls and including means (22, 40) for protecting the cutaneous zone treated.

2. A packaging according to claim 1, **characterised in that** the protection means of the cutaneous zone treated consist of a bandage, a compress, a plaster or a dressing (22, 40).

3. A packaging according to claim 1, **characterised in that** the or each wall (1, 12, 13, 33, 34) of the first pocket (3, 11, 31) whereon the impregnated support (2, 14, 32) is secured, is made of a flexible material.

4. A packaging according to claim 1, **characterised in that** two pockets (11, 17, 31, 35, 39) at least are superimposed on top of one another, so that two successive pockets are connected by a common wall (12, 34, 38).

5. A packaging according to claim 1, **characterised in that** means are provided in one or the pockets intended to receive a sterile content, for guaranteed sterility of said content during storage.

6. A packaging according to claim 1, **characterised in that** the fastening means of the walls (1, 12, 13, 18, 33, 34, 37, 38) of each pocket (3, 11, 17, 31, 35, 39) enable separation by peeling said walls when opening the pocket considered.

7. A packaging according to claim 1, **characterised in that** antirupture means (2, 14, 32, 35) are provided on at least the first pocket (3, 11, 31) to prevent, when opening the pocket, the walls (1, 12, 13, 33, 34) forming said pocket from being disconnected totally.

8. A packaging according to the previous claim, **characterised in that** the antirupture means of the pockets (3, 11, 31, 35) containing an impregnated support (2, 14, 32, 35) consist essentially in securing said impregnated support to both walls of the pocket containing said support.

9. A packaging according to claim 1, **characterised in that** one or each pocket (3, 11, 17, 31, 35, 39) includes opening means (6, 7, 15, 16, 20, 21, 41, 42, 43, 44, 45) for easier opening thereof.

10. A packaging according to claims 7 to 9, **characterised in that** the antirupture and/or the opening means (6, 7, 15, 16, 20, 21, 41, 42, 43, 44, 45) of a pocket are situated offset or opposite relative to the antirupture means and/or the opening means of the following pocket, respectively, on the wall common to both pockets.
